# EUROPEAN PATENT SPECIFICATION

(11) **EP 2 977 000 B1**
(45) Date of publication and mention of the grant of the patent: **17.05.2017**
(21) Application number: 15177907.1
(22) Date of filing: 22.07.2015
(51) Int. Cl.: A61B 8/08, A61F 9/007, A61B 5/03, A61B 5/00, A61B 8/10, A61B 8/00

(54) **HEAD FRAME WITH INTEGRATED PRESSURE CHAMBER FOR NON-INVASIVE INTRACRANIAL PRESSURE MEASUREMENTS**
KOPFRAHMEN MIT INTEGRIERTER DRUCKKAMMER FÜR NICHTINVASIVE INTRAKRANIALE DRUCKMESSUNGEN
CADRE DE TÊTE À CHAMBRE DE PRESSION INTÉGRÉE POUR DES MESURES DE LA PRESSION INTRACRÂNIENNE NON INVASIVES

(30) Priority: 24.07.2014 US 201414339982
(43) Date of publication of application: 27.01.2016
(73) Proprietor: UAB Vittamed, 51362 Kaunas (LT)
(72) Inventor: RAGAUSKAS, Arminas, 51362 Kaunas (LT)
(74) Representative: Patentgruppen A/S

(56) References cited:
- US-A- 3 903 871
- US-A- 5 951 477
- US-A1- 2010 331 684
- US-A1- 2013 238 015

## Description

### FIELD OF THE INVENTION

The present invention generally relates to an apparatus for non-invasively measuring intracranial pressure and more specifically relates to a head frame with an integrated pressure chamber for non-invasively measuring intracranial pressure.

### BACKGROUND OF THE INVENTION

This invention is a new apparatus capable of being used in conjunction with our methods previously described in U.S. Pat. Nos. 5,951,477 and 8,394,025. This new head frame offers advantages over the apparatuses described in the '477 and '025 patents.

An apparatus for determining the pressure and flow inside the ophthalmic artery is described in U.S. Pat. No. 4,907,595 to Strauss. The apparatus uses a rigid chamber that can be affixed and sealed over the human eye so that it can be pressurized to apply an external pressure against the eyeball. An ultrasonic transducer is also mounted to the chamber and oriented to transmit ultrasonic pulses for a Doppler type measurement of the flow inside the ophthalmic artery (OA). The apparatus operates by enabling an operator to increase the pressure to such a level that the blood flow through the OA ceases. The pressure at which this occurs is then an indication of the pressure inside the OA. Typically, the pressure at which this event occurs is in the range of about 170 mmHg.

A problem associated with an apparatus as described in the '595 Patent is that the pressure necessary to obtain the desired measurement is so high that it generally exceeds maximum recommended pressures by a significant amount. When such device is then used for an extended time, tissue damage can arise and may result in an increase in the intracranial pressure, ICP, to unacceptable levels.

In our previously granted '477 and '025 patents we described an apparatus and method for determining the pressure inside the brain. An apparatus as described in the '477 and '025 patents requires annular inflatable pressure chamber with an ultrasonic transducer positioned in the open center of the annular chamber. Thus, the ultrasonic transducer would be positioned against the closed eye-lid of a patient, only separated by the thin, flexible layer of ultrasonically transparent material, sonogel or sonopad.

An apparatus in accordance with either the '477 or the '025 patents restricted the size of the ultrasonic transducer. The diameter of the ultrasonic transducer could only be equal to or less than the diameter of the opening in the annular pressure chamber. The ultrasonic transducer was further limited in size and movement by skull bones around the eye. Further, previously described apparatuses allowed for risk of injury to the patient's eye or facial tissues arround the eye upon movement and adjustment of the ultrasonic transducer's position either manually or robotically. Furthermore, an apparatus with similar features is known from U.S. Pat. No. 3,903,871.

### SUMMARY OF THE INVENTION

An apparatus in accordance with the invention is a head frame , as defined in claim 1. It further comprises a shield with a smooth, hard surface and a pressure controlled ultrasonically transparent liquid-filled chamber formed by an elastic film fixed to one side of the lens or shield. Connectors positioned on the lens allow for inlet and outlet of liquid into the pressure chamber. When the head frame of the invention is mounted on the head of a patient and pressure in the chamber is increased, the elastic film expands and conforms to shape of the patient's closed eye imparting a pressure on the tissues around the eye and orbital tissues.

With an apparatus in accordance with the invention one can derive an indication of the pressure inside a skull in a non-invasive manner using previously known methods, such as that described in or '025 patent without risk of injury to the patient's eye. Such methods involve use of an ultrasonic Doppler device to measure blood flow velocities in intracranial and extracranial segments of the ophthalmic artery under varying amounts of pressure applied to the tissues around the eye.

An apparatus in accordance with the invention allows for measurement of the intracranial pressure of a patient without placing the ultrasonic transducer of Doppler device against the eye-lid of the patient. A further aspect of the invention enables the use of a wide range of ultrasonic transducers of different sizes. The diameter of the ultrasonic transducer of Doppler device is not limited, allowing for optimization of the ultrasonic beam and better Doppler signal to noise ratio. Because the ultrasonic transducer of Doppler device is not placed against the eye of the patient, the present invention enables measurement of intracranial pressure by manual operator or robotic driver without discomfort or risk of injury to patient.

An apparatus in accordance with the invention is compatible with all patients and can conform to any patent's eye independent of race and/or facial structure. An apparatus in accordance with the invention provides a surface area that allows for the ultrasonic transducer of Doppler device to be positioned away from the eye and allows for free movement across the entire area of the shield or lens.

It is therefore an object of the invention to allow for the fast and safe optimal positioning of the ultrasonic transducer in order to cross the intracranial and extracranial segments of an ophthalmic artery with the ultrasonic beam at the needed depths from the surface of the ultrasonic transducer and also for non-invasive measurement of intracranial pressure of any patient regardless of age, race, or facial structure.

### BRIEF DESCRIPTION OF THE DRAWINGS

FIG. 1 is a perspective view of the apparatus positioned on the head of a patient.
FIG. 2 is an exploded view of the head frame.
FIGS. 3a - 3b are perspective views of the head frame with a collapsed chamber and an expanded chamber, respectively.
FIGS. 4a - 4c are front elevational view, top plan view and a side elevational view with the dotted line indicating an expanded chamber, respectively.
FIG. 5 is a right side cutaway view of the head frame positioned on the head of a patient showing the head frame in use with a transducer, the dotted line indicating the elastic film of the expanded chamber.

### DETAILED DESCRIPTION OF THE INVENTION

The exemplary embodiments of the present invention may be further understood with reference to the following description and the related appended drawings, wherein like elements are provided with the same reference numerals. The exemplary embodiments of the present invention are related to a head frame for use in non-invasively determining the absolute value of intracranial pressure (ICP) of a living body.

With an apparatus in accordance with the invention the ICP inside a person's head can be determined from an observation of the blood velocities inside the two segments of the ophthalmic artery (OA) by using an ultrasonic Doppler apparatus which senses the response of the blood flow to a pressure "challenge" applied to the tissues around the eye and orbital tissues. The pressure is applied to the eye at the necessary level for equilibrating parameters representative of the intracranial and extracranial blood flows in the OA leading to the eye. The possibility of this type of measurement has been demonstrated with the analysis presented in our previous U.S. Patent Nos. 5,951,477 and 8,394,025.

The apparatus of the present invention is significantly improved over previous apparatuses used for noninvasive measurement of ICP for several reasons. The transducer surface of an apparatus in accordance with the present invention is not located against the closed eyelid of a patient, but is located on the shield of the apparatus, reducing risk of injury when an ultrasonic device is being used to take measurements.

Additionally, the shield provides an unrestricted surface to allow for easy movement and manipulation of ultrasonic transducer. Manipulation of the ultrasonic transducer of Doppler device is required to locate and steer the ultrasonic transducer toward the intracranial and extracranial segments of of the ophthalmic artery. The manipulation and steering of the ultrasonic transducer may be performed manually or by robotic steering. Because the transducer is not placed against the eyelid of the patient, robotic manipulation is much safer with the present invention.

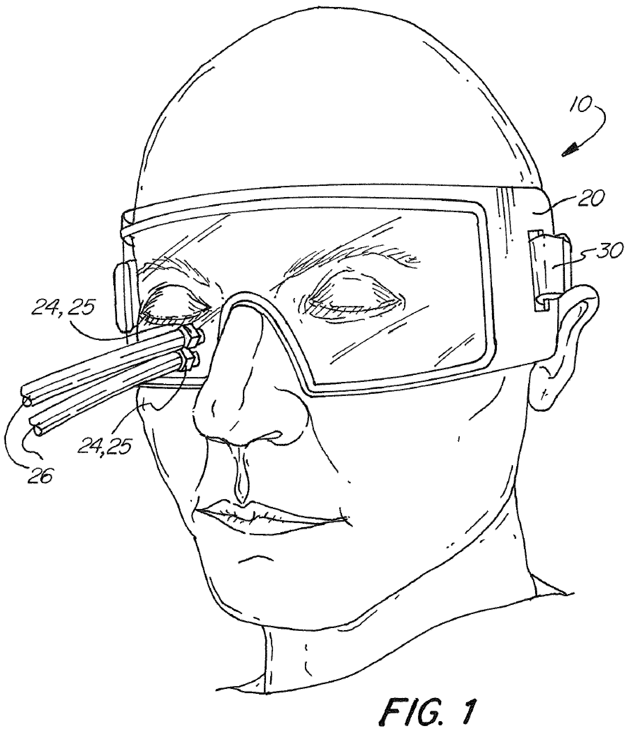

With reference to Figure 1, a head frame 10 is shown positioned on the head of a patient. Lens or shield 20 is configured to fit over at least an eye area of the patient. Shield 20 in some embodiments extends across the wearer's face from one temple, over the bridge of the nose to the other temple. Shield 20 extends vertically along the wearer's face from about the cheek bone or lower wall of the orbital to about the upper wall of the orbital or the brow of the wearer. In some embodiments not according to the invention the shield 20 extends only over one eye of the patient.

Strap 30 secures head frame 10 in place on the head of the patient. Elastic film 22 is fixed to the inner side of the shield 20. An expandable chamber 28 is formed by the elastic film 22 sealed with the shield 20. To inflate the chamber 28, pressurized liquid is sent into the chamber through a first connector 24. When the chamber 28 is pressurized the elastic film 22 conforms to the eye of a patient and imparts pressure on the eye and tissues around the eye of the patient. Connectors 24, 25 provide an inlet and outlet of liquid into the chamber 28 for adjusting the pressure of the chamber 28 and are in connection with a pressurized liquid. The location of the connectors 24, 25 may be anywhere on the shield that would not interfere with the manipulation of a transducer in measuring or monitoring the ICP of the patient. In the Figures, the connectors 24, 25 are located near the edge of the shield close to the nose of the patient, but could be positioned in other locations.

The connectors 24, 25 are in connection with a source of pressurized liquid. The pressurized liquid may be any ultrasonically transparent liquid. It is preferable that the liquid have low attenuation of ultrasound and provide optimal speed of ultrasound. In some embodiments the pressurized liquid is water. Pure water without gas is the preferred liquid. The pressurized liquid may also be an acoustic gel. Ultrasonically transparent liquid is liquid that does not contain solid particles or gas. It is further preferable that the pressurized liquid does not pose a hazard to the patient in the case of a leak or rupture.

In some embodiments connectors 24, 25 are connected via tubing 26 to a system for monitoring and adjusting the amount of liquid and therefore pressure in the chamber 28. For example, the tubing may be connected to an electromechanical pump, valves, microcontroller with pressure sensor, and other components to monitor and adjust the pressure in the chamber 28.

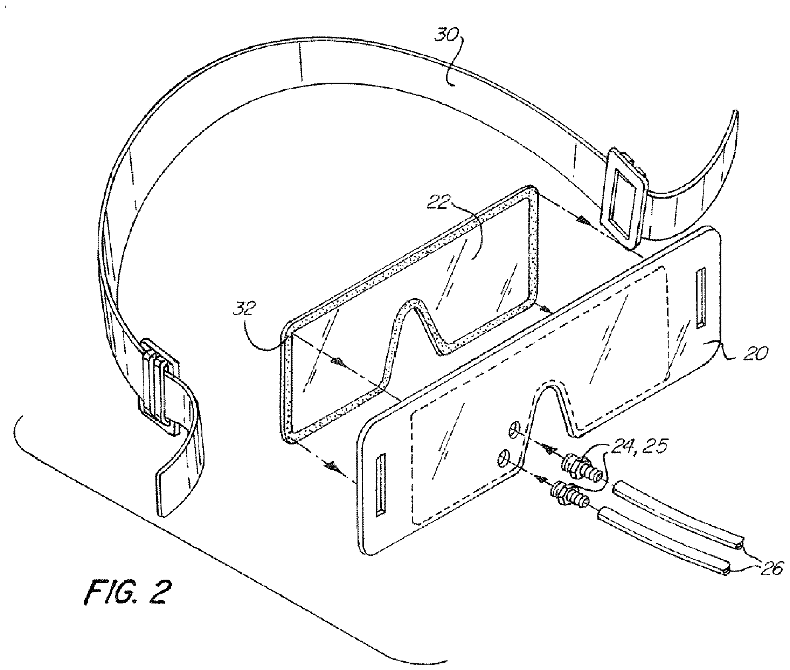

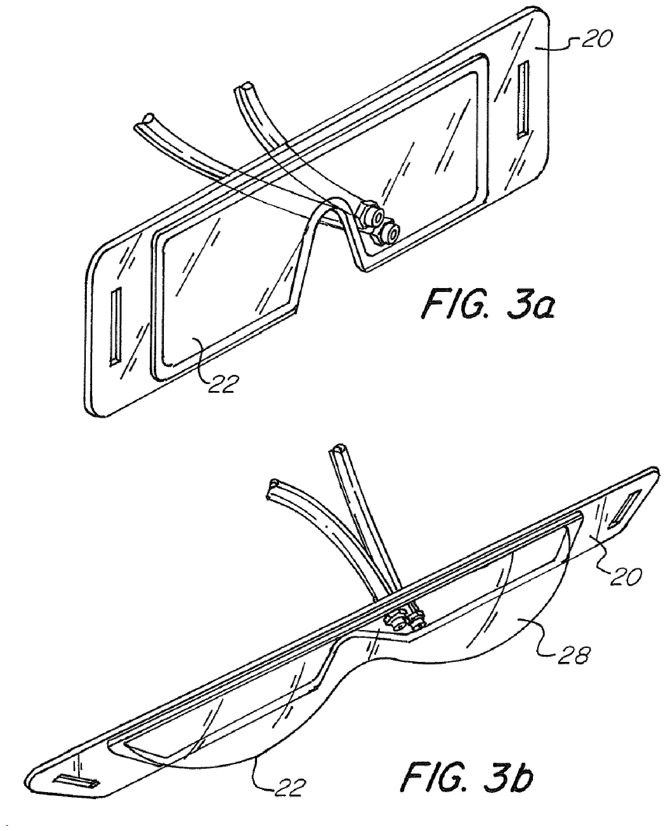

As best seen in Figure 2, an exploded view of head frame 10 is shown. Shield 20 is configured to cover at least an eye area of a patient. First and second connectors 24, 25 are positioned on shield 20. Elastic film 22 is fixed to shield 20 and expands when pressurized forming a chamber 28 (See Fig. 3a-3b). In some embodiments elastic film 22 is fixed to shield 20 along its outer edge 32 by liquid adhesive. In some embodiments elastic film 22 is fixed to shield 20 in such a way as to form a hermetic seal to prevent liquid from escaping chamber 28 when pressurized up to 100 mmHg.

The strap 30 of the head frame 10 may be elastic or inelastic. The strap 30 is adjustable so as to allow the head frame 10 to be used with any patient. The head frame 10 of this invention may be disposable.

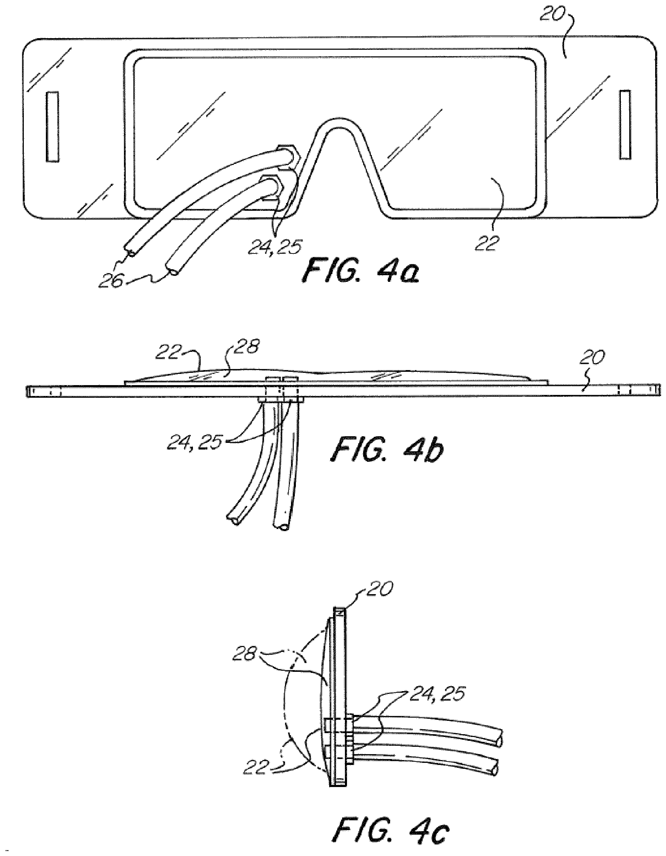

As can be seen best in the series of Figures 3 and 4 elastic film 22 flexes and chamber 28 expands with the application of pressure/introduction of liquid. The connectors 24, 25 allow for the inlet and outlet of liquid to and from the chamber 28. The expanded chamber 28 applies pressure to a patient's eye when head frame 10 is positioned on the head of a patient. Elastic film 22 conforms to the shape of the eye when the chamber 28 is pressurized imparting a slight pressurization of the tissues around the eye. These tissues are contiguous with tissues in the posterior orbital portion of the eye socket, so the applied pressure is effective there as well. This results in a pressurization of the extracranial segment of an ophthalmic artery.

It is preferable that in combination, the materials of shield 20 and elastic film 22 must not distort or attenuate an ultrasonic beam in the frequency range 1.5 MHz to 3.0 MHz. It is further preferable that the materials be non-allergenic and transparent. The shield 20 of the current invention provides a suitable surface for making acoustic contact with an ultrasonic transducer 40. In some embodiments shield 20 is made of transparent polycarbonate. The elastic film 22, in some embodiments is made of synthetic polyisoprene latex having a thickness between 40 and 60 microns.

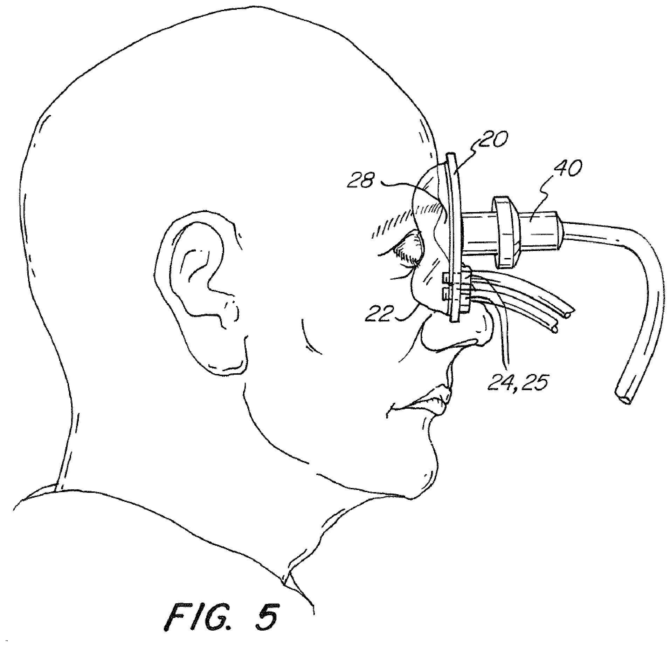

Figure 5 illustrates the location of the transducer away from the patient's eye. The transducer 40 is manipulated on the transducer surface of the shield 20. Figure 5 is also the best illustration of the elastic film 22 conforming to the eye of the patient upon inflation of the chamber 28.

An advantage of head frame 10 is that the structure of the head frame 10, with corresponding lens 20 and chamber 28, provides protection for the patient's eye when an ultrasonic transducer of Doppler device or transducer 40 is in use. The apparatus of this invention further allows for safe manual or robotic positioning of ultrasonic transducer and for measurement of intracranial pressure without risk of injury to patient. Head frame 10 allows for accurate measurements by an ultrasonic Doppler device by providing a lens 20 surface that can accommodate a variety of ultrasonic transducer diameters. Ultrasonic transducers used in connection with the invention are preferably between 15mm and 30mm in diameter.

By accommodating a wide range of transducer sizes, the apparatus of this invention provides the user with the ability to optimize the diameter of the ultrasonic transducer 40 to achieve the most accurate Doppler signals. Larger diameter transducers provide a greater ability to focus the ultrasonic beam. A focused ultrasonic beam provides stronger signals and better signal to noise ratios.

For use with a patient, the operator or user would place the head frame 10 on the head of the patient with the elastic film 22 proximal the patient's eye area. The operator would adjust the strap 30 to secure the head frame 10 in place. The operator would then apply pressure to the eye of the patient by sending pressurized liquid through the first connector 24. The elastic film 22 would expand and conform to the eye of the patient.

Next, the operator would position the transducer in acoustic contact with the shield 20 for either manual or robotic manipulation. The positions and optimal depths of intracranial and extracranial segments of the ophthalmic artery are located by manipulation of the transducer 40 on the outer surface of the shield 20 away from the patient's eye. The transducer 40 connected to Doppler device can then measure the velocity of the blood flow in the intracranial and extracranial segments of the OA. The operator may adjust the pressure by sending fluid in through connector 24 or allowing fluid out of the chamber 28 through a second connector 25. The adjustment of pressure in the chamber 28 has little or no effect on the angle and position of the ultrasonic transducer 40. This allows the transducer 40 to continue measuring the appropriate blood flow velocities as the pressure is adjusted.

It should be understood that the foregoing is illustrative and not limiting, and that obvious modifications may be made by those skilled in the art without departing from the scope of the claims, which define the invention.

## Claims

1. A head frame (10) for use in non-invasively determining the absolute value of intracranial pressure of a living body such as a patient comprising:
a hard, flexible shield (20) adapted to cover an eye area of the patient,
wherein the shield (20) comprises an inner surface and an outer transducer surface;
an elastic film (22) having an outer edge (32), wherein said elastic film is sealed along its outer edge to the inner surface of the shield (20) forming an expandable chamber (28);
a first connector (24) located on the shield (20) adapted to permit an inflow of pressurized liquid into said chamber (28);
wherein said elastic film (22) is adapted to expand inward toward the patient's eye with an inflow of pressurized liquid into said chamber (28) imparting a pressure against the eye of the patient; and
an adjustable strap (30) attached to the ends of said shield (20) adapted to secure the head frame (10) to the patient;
**characterized in that** the head frame (10) comprises a second connector (25) located on the shield (20) adapted to permit an outflow of pressurized liquid from the chamber (28); and **in that** the hard, flexible shield (20) and the elastic film (22) are adapted for covering the eye area of the patient extending from the right temple over the bridge of the nose to the left temple, and extending from a point below the lower wall of the patient's orbital to a point above the upper wall of the patient's orbital.

2. The head frame of claim 1, wherein the elastic film (22) is fixed to the shield (20) by a liquid adhesive.

3. The head frame of any one of claims 1-2, wherein the chamber (28) is hermetically sealed under pressures up to 100 mmHg.

4. The head frame of any one of claims 1-3, wherein the outer transducer surface is smooth and suitable for making acoustic contact with an ultrasonic transducer.

5. The head frame of any one of claims 1-4, wherein the shield (20) is 1.0-2.0 mm polycarbonate.

6. The head frame of any one of claims 1-5, wherein the elastic film (22) conforms to the outer surface of the patient's closed eye when the chamber (28) is pressurized.

7. A method for noninvasively determining the absolute value of intracranial pressure of a living body such as a patient comprising:
providing a head frame (10), according to claim 1;
placing said head frame (10) on the patient wherein the elastic film (22) is positioned proximal the patient;
applying external pressure to the eye of the patient by sending pressurized liquid through the first connector (24) inflating said chamber (28); and
placing an ultrasonic transducer (40) in acoustic contact with said outer surface of said shield (20) to noninvasively locate an intracranial segment and extracranial segment of an ophthalmic artery extending from inside the cranium into the eye by steering the ultrasonic transducer (40) to measure the velocity of blood flow at a plurality of depths through the pressurized chamber (28).

8. The method of claim 7, wherein the steering of the ultrasonic transducer (40) is performed by robotic means.

9. The method of claim 7 or 8, further comprising adjusting the external pressure applied to the eye by sending pressurized liquid through the first connector (24) and permitting the outflow of pressurized liquid from the chamber (28) through the second connector (25).

## Patentansprüche

1. Kopfgestell (10) zur Verwendung bei der nicht-invasiven Bestimmung des absoluten Werts des Hirndrucks eines lebenden Körpers, wie etwa eines Patienten, Folgendes umfassend:
einen harten, biegsamen Schirm (20), der dafür eingerichtet ist, einen Augenbereich des Patienten abzudecken,
wobei der Schirm (20) eine innere Fläche und eine äußere Wandlerfläche umfasst;
eine elastische Folie (22), die einen Außenrand (32) hat, wobei die elastische Folie, die entlang ihrem Außenrand zur inneren Fläche des Schirms (20) abgedichtet ist, ein expandierbare Kammer (28) bildet;
einen ersten Anschluss (24), der sich auf dem Schirm (20) befindet und der dafür eingerichtet ist, einen Zufluss einer unter Druck stehenden Flüssigkeit in die Kammer (28) zu gestatten;
wobei die elastische Folie (22) dafür eingerichtet ist, mit einem Zufluss von unter Druck stehender Flüssigkeit in die Kammer (28) nach innen in Richtung des Auges des Patienten zu expandieren, wodurch ein Druck auf das Auge des Patienten ausgeübt wird; und
ein einstellbares Halteband (30), das an den Enden des Schirms (20) befestigt ist und das dafür eingerichtet ist, das Kopfgestell (10) am Patienten zu fixieren;
**dadurch gekennzeichnet, dass** das Kopfgestell (10) einen zweiten Anschluss (25) umfasst, der sich auf dem Schirm (20) befindet und der dafür eingerichtet ist, einen Abfluss einer unter Druck stehenden Flüssigkeit aus der Kammer (28) zu gestatten; und dadurch, dass
der harte, biegsame Schirm (20) und die elastische Folie (22) dafür eingerichtet sind, den Augenbereich des Patienten abzudecken, der sich von der rechten Schläfe über die Nasenbrücke zur linken Schläfe erstreckt und der sich von einem Punkt unterhalb der unteren Wand der Augenhöhle des Patienten zu einem Punkt oberhalb der oberen Wand der Augenhöhle des Patienten erstreckt.

2. Kopfgestell nach Anspruch 1, wobei die elastische Folie (22) am Schirm (20) durch einen Flüssigkleber befestigt ist.

3. Kopfgestell nach einem der Ansprüche 1 bis 2, wobei die Kammer (28) unter Drücken von bis zu 100 mm Hg hermetisch abgedichtet ist.

4. Kopfgestell nach einem der Ansprüche 1 bis 3, wobei die äußere Wandlerfläche glatt ist und dafür geeignet ist, mit einem Ultraschallwandler einen akustischen Kontakt herzustellen.

5. Kopfgestell nach einem der Ansprüche 1 bis 4, wobei der Schirm aus 1,0 bis 2,0 mm Polycarbonat besteht.

6. Kopfgestell nach einem der Ansprüche 1 bis 5, wobei die elastische Folie (22) sich der Außenfläche des geschlossenen Auges des Patienten anpasst, wenn die Kammer (28) unter Druck steht.

7. Verfahren zur nicht-invasiven Bestimmung des absoluten Werts des Hirndrucks eines lebenden Körpers, wie etwa eines Patienten, Folgendes umfassend:
Bereitstellen eines Kopfgestells (10) nach Anspruch 1;
Platzieren des Kopfgestells (10) am Patienten, wobei die elastische Folie (22) zum Patienten hin positioniert wird;
Anwenden eines externen Drucks auf das Auge des Patienten, indem eine unter Druck stehende Flüssigkeit durch den ersten Anschluss (24) geleitet wird, wodurch sich die Kammer (28) aufpumpt; und
Platzieren eines Ultraschallwandlers (40) in akustischem Kontakt mit der äußeren Fläche des Schirms (20) zur nicht-invasiven Lokalisierung eines intrakraniellen Segments und eines extrakraniellen Segments einer Augenarterie, die vom Inneren des Schädels in das Auge hinein verläuft, indem der Ultraschallwandler (40) gesteuert wird, um die Geschwindigkeit des Blutflusses in mehreren Tiefen durch die unter Druck stehende Kammer (28) zu messen.

8. Verfahren nach Anspruch 7, wobei die Steuerung des Ultraschallwandlers (40) durch ein Robotermittel ausgeführt wird.

9. Verfahren nach Anspruch 7 oder 8, das außerdem das Einstellen des externen Drucks umfasst, der auf das Auge angewendet wird, indem unter Druck stehende Flüssigkeit durch den ersten Anschluss (24) geleitet wird und indem der Abfluss von unter Druck stehender Flüssigkeit aus der Kammer (28) durch den zweiten Anschluss (25) gestattet wird.

## Revendications

1. Cadre de tête (10) pour une utilisation dans la détermination non invasive de la valeur absolue de pression intracrânienne d'un corps vivant tel qu'un patient comprenant :
un écran de protection dur flexible (20) adapté pour couvrir une zone oculaire du patient,
dans lequel l'écran de protection (20) comprend une surface interne et une surface de transducteur externe ;
un film élastique (22) comportant un bord externe (32), dans lequel ledit film élastique est scellé le long de son bord externe à la surface interne de l'écran de protection (20) formant une chambre extensible (28) ;
un premier raccord (24) situé sur l'écran de protection (20) adapté pour permettre une arrivée de liquide sous pression dans ladite chambre (28) ;
dans lequel ledit film élastique (22) est adapté pour s'étendre vers l'intérieur en direction de l'oeil du patient, une entrée de liquide sous pression dans ladite chambre (28) appliquant une pression contre l'oeil du patient ; et
une sangle réglable (30) attachée aux extrémités dudit écran de protection (20) adaptée pour attacher le cadre de tête (10) au patient ;
**caractérisé en ce que** le cadre de tête (10) comprend un second raccord (25) situé sur l'écran de protection (20) adapté pour permettre une sortie de liquide sous pression de la chambre (28) ; et **en ce que** l'écran de protection dur flexible (20) et le film élastique (22) sont adaptés pour couvrir la zone oculaire du patient s'étendant de la tempe droite, au-dessus de la voûte nasale, jusqu'à la tempe gauche, et s'étendant d'un point en dessous de la paroi inférieure de l'orbite du patient à un point au-dessus de la paroi supérieure de l'orbite du patient.

2. Cadre de tête selon la revendication 1, dans lequel le film élastique (22) est fixé à l'écran de protection (20) par un adhésif liquide.

3. Cadre de tête selon l'une quelconque des revendications 1 et 2, dans lequel la chambre (28) est fermée hermétiquement à des pressions allant jusqu'à 100 mmHg.

4. Cadre de tête selon l'une quelconque des revendications 1 à 3, dans lequel la surface du transducteur externe est lisse et appropriée pour établir un contact acoustique avec un transducteur ultrasonore.

5. Cadre de tête selon l'une quelconque des revendications 1 à 4, dans lequel l'écran de protection (20) est du polycarbonate de 1,0 à 2,0 mm.

6. Cadre de tête selon l'une quelconque des revendications 1 à 5, dans lequel le film élastique (22) épouse la surface externe de l'oeil fermé du patient lorsque la chambre (28) est sous pression.

7. Procédé de détermination non invasive de la valeur absolue de la pression intracrânienne d'un corps vivant tel qu'un patient comprenant :
la fourniture d'un cadre de tête (10), selon la revendication 1 ;
le placement dudit cadre de tête (10) sur le patient dans lequel le film élastique (22) est positionné près du patient ;
l'application de pression externe sur l'oeil du patient par l'envoi de liquide sous pression à travers le premier raccord (24) gonflant ladite chambre (28) ; et
la mise en contact acoustique d'un transducteur ultrasonore (40) avec ladite surface externe dudit écran de protection (20) pour localiser de manière non invasive un segment intracrânien et un segment extracrânien d'une artère ophtalmique s'étendant de l'intérieur du crâne jusque dans l'oeil en orientant le transducteur ultrasonore (40) pour mesurer la vitesse du débit sanguin à une pluralité de profondeurs à travers la chambre sous pression (28).

8. Procédé selon la revendication 7, dans lequel l'orientation du transducteur ultrasonore (40) est effectuée à l'aide de moyens robotiques.

9. Procédé selon la revendication 7 ou 8, comprenant en outre le réglage de la pression externe appliquée sur l'oeil en envoyant du liquide sous pression à travers le premier raccord (24) et en permettant la sortie de liquide sous pression de la chambre (28) à travers le second raccord (25).
